# EUROPÄISCHE PATENTSCHRIFT

(11) **EP 0 555 446 B1**
(45) Veröffentlichungstag und Bekanntmachung des Hinweises auf die Patenterteilung: **28.07.1999**
(21) Anmeldenummer: 92918251.7
(22) Anmeldetag: 28.08.1992
(51) Int. Cl.: A61K 31/495

(54) **VERWENDUNG VON ANTAGONISTEN ODER PARTIELLEN AGONISTEN AM 5-HT 1a-REZEPTOR ZUR BEHANDLUNG UND PRÄVENTION VON KOGNITIVEN STÖRUNGEN**
USE OF ANTAGONISTS OR PARTIAL AGONISTS ON THE 5-HT 1a RECEPTOR FOR THE TREATMENT AND PREVENTION OF COGNITIVE DISORDERS
UTILISATION D'ANTAGONISTES OU D'AGONISTES PARTIELS SUR UN RECEPTEUR 5-HT 1a POUR LE TRAITEMENT ET LA PREVENTION DE TROUBLES COGNITIFS

(30) Priorität: 31.08.1991 DE 4135551
(43) Veröffentlichungstag der Anmeldung: 18.08.1993
(73) Patentinhaber: BAYER AG, 51368 Leverkusen (DE)
(72) Erfinder: TURNER, Jonathan, D-1000 Berlin 28 (DE); COLE, Belinda, D-1000 Berlin 12 (DE)
(86) Internationale Anmeldenummer: DE9200720
(87) Internationale Veröffentlichungsnummer: WO9304681

(56) Entgegenhaltungen:
- EP-A- 390 424
- EP-A- 0 338 989
- DE-A- 4 039 631
- GB-A- 1 567 845
- GB-A- 2 222 768
- US-A- 4 423 049
- Dialog Information Services, File 73, Embase Dialog accession No. 8029555, Embase accession No. 91057805, Murphy D.L.: " Neuropsychiatric disorders and the multiple human brain serotonin receptor subtypes and subsystems"; & Neuropsychopharmacology (USA), 1990, 3/5-6 (457-471)
- Dialog Information Services, File 155, Medline, Dialog accession No. 07610664, Medline accession No. 91129664, Rydelek-Fitzgerald L et al: "NAN-190: agonist and antagonist interactions with brain 5-HT1A receptors "; & Brain Res. Nov. 5 1990, 532 (1-2) p 191-6

## Beschreibung

Die Erfindung betrifft die neue Verwendung von Antagonisten oder partiellen Agonisten am 5-HT₁ₐ-Rezeptor oder deren physiologisch verträgliche Salze zur Herstellung eines Arzneimittels zur Prävention und Behandlung von kognitiven Störungen.

Serotonin (5-Hydroxytryptamin, 5-HT) ist ein Neurotransmitter, der von bestimmten neuronalen Zellgruppen freigesetzt wird, die sich im Mittelhirn befinden und die das gesamte Gehirn innervieren. Der aus den Neuronen freigesetzte aminerge Neurotransmitter Serotonin besitzt modulatorische Wirkungen auf Zielstrukturen, wobei die Wirkungen von pharmakologisch unterschiedlichen Rezeptoren vermittelt werden. Diese Rezeptoren werden wie folgt klassifiziert: 5-HT₁ - ähnlich mit Subtypen a-e, 5-HT₂-, 5-HT₃- und 5-HT₄-Rezeptoren. Von den bekannten 5-HT₁-Rezeptoren ist der 5-HT₁ₐ-Subtyp bisher am weitesten untersucht, da mit 8-Hydroxydipropylamino-tetralin (8-OH-DPAT) ein selektiver Agonist zur Verfügung steht.

Aus EP-A-0041488 ist bekannt, daß bei Krankheiten, die auf Serotonin-Mangel beruhen, wie senile Störungen der mentalen Funktion beispielsweise Senile Demenz, 5-HT₁ₐ-Agonisten eingesetzt werden können. Ferner wird in EP-A-0345 948 beschrieben, daß 5 HT₁ₐ-Agonisten wie 8-OH-DPAT zur Behandlung von Cerebraler Ischämie nach Herzstillstand oder Schlaganfall und zur Behandlung von Multiinfarkt Demenz geeignet sind.

Unter 5HT₁ₐ-Agonisten versteht man Verbindungen, die an den 5-HT₁ₐ-Rezeptor binden und die biologische Antwort auslösen. Unter 5-HT₁ₐ-Antagonisten sind Verbindungen zu verstehen, die an den 5-HT₁ₐ-Rezeptor binden und die biologische Antwort blockieren. Unter 5-HT₁ₐ-Partialagonisten sind Verbindungen zu verstehen, die den 5-HT₁ₐ-Rezeptor direkt aktivieren, aber einen schwächeren max. Effekt auslösen.

Überraschenderweise wurde gefunden, daß Antagonisten oder partielle Agonisten an 5-HT₁ₐ-Rezeptoren eine Verbesserung der kognitiven Leistung hervorrufen.

Aufgrund ihres Wirkungsmechanismus eignen sich Antagonisten und partielle Agonisten am 5-HT₁ₐ-Rezeptor zur Behandlung und Prävention von kognitiven Störungen die bei älteren Menschen auftreten können oder bei der Alzheimerschen Krankheit, bei der Parkinson Krankheit mit assoziierter kognitiver Beeinträchtigung, bei AIDS-bezogener Demenz und anderen kognitiven Beeinträchtigungen.

Erfindungsgemäß geeignet sind partielle 5-HT₁ₐ-Rezeptor-Agonisten, die eine intrinsische Aktivität kleiner als die von 5-Carboxamidotryptamin besitzen und 5-HT₁ₐ-Rezeptor-Antagonisten, d.h. Verbindungen, die an den 5-HT₁ₐ-Rezeptor binden und die von Agonisten hervorgerufene Wirkung antagonisieren.

Die Methoden zur Bestimmung der 5-HT₁ₐ-antagonistischen bzw. agonistischen Aktivität werden beispielsweise in J. Pharmacol. Exp. Ther. 238, 248-253_(1986) und J. Pharmacol. Exp. Ther. 258, 58 - 65 (1991) beschrieben.

Als synthetisch chemische Verbindungen, die die erfindungsgemäße Wirkung zeigen, seien beispielsweise die folgenden Kassen von partiellen 5-HT₁ₐ-Rezeptor-Agonisten und 5-HT₁ₐ-Rezeptor-Antagonisten genannt:
1.) Piperazinderivate wie 1,2-Methoxyphenyl-4-[4-(2-phtalamidbutyl]-piperazin-Hydrochlorid (NAN-190) *z.B. Rydelek-Fitzgerald et al., Brain Res. **532** 191-196, 1990)*; 2-[4-[4-(2-Pyrimidinyl)-1-piperazinyl]butyl]-1,2-benzisothiazolin-3-on-1,1-dioxid (Ipsapiron) (*z.B. O'Connor et al*., *Brit. J. Pharmacol. **101** 171-177, 1990);N-[4-[4-(2-Pyridinyl)-1-piperazinyl]butyl]-1,1-cyclopentanediacetamid (*Buspiron) *(z.B. deVivo and Maayani, J. Pharmacol. Exp. Ther. **238** 248-253, 1986);* 8-[2-[4-(2-Methoxyphenyl)-1-piperazinyl)ethyl]-8-azaspiro [4,5]-decan-7,9-dion (BMY 7378, *z.B. Yocca et al., Eur. J. Pharmacol. **137** 293-294, 1987);* 3,3-Dimethyl-1-[4-[4-(2-pyrimidinyl)-1-piperazinyl]butyl]glutarimid)*(*Gepiron) (z.B. *Eison et al., Pharmacol. Biochem. Behav. 24 **701**, 1986*) 1-[10,11-Dihydro-8-(methylthio)dibenzo[b,f]thiepin-10-yl]-4-methylpiperazin (Methiothepin).
2.) Benzodioxane wie 8-(4-[(1,4-Benzodioxan-2-ylmethyl)amino]butyl]-8-azaspiro[4,5]decan-7,9-dion (MDL 72832 *z.B. Hibert et al., J. Med. Chem. 31 1087-1093, 1988*; 8[2-[(2,3-Dihydro-1,4-Benzodioxin-2-yl)methylamino]ethyl]-8-azaspiro[4,5]decan-7,9-dion (MDL 73005 EF, *z.B. Hibert, M. et al., Brit. J. Pharmacol. 93 Suppl. 2P, 1988; Moser, P. et al. ibid 3P).*
3.) 1,1-Diethyl-3-(8a-ergolinyl)-harnstoffderivate wie Lisurid (*z.B. die Vivo and Maayani, J. Pharmacol. Exp. Ther. 238 248-253*) und Tergurid. (z.B. *Kehr, EurJ. Pharmacol. 97.111-119, 1984.*)
4.) 2-Amino-tetraline wie 5-Fluor-8-hydroxy-2-(dipropylamino)-tetralin (J. Med. Chem. 1990, 33, 1541-1544) und Spiperon (8-[3-(p-Fluorbenzoyl)propyl]-1-phenyl-1,3,8-triazaspiro[4,5]decan-4-on).

Die physiologisch verträglichen Säureadditionssalze der obigen Wirkstoffe sowie alle möglichen Isomeren (Stereoisomeren und/oder Enantiomeren) und deren Gemische können erfindungsgemäß verwendet werden.

Die physiologisch verträglichen Salze leiten sich von Alkali- oder Erdalkalimetallen oder den üblichen anorganischen oder organischen Säuren wie Salzsäure, Bromwasserstoffsäure, Schwefelsäure, Phosphorsäure, Zitronensäure, Maleinsäure oder Fumarsäure ab. Die erfindungsgemäßen Wirkungen werden am Beispiel von 1,2-Methoxyphenyl-4-[4-(2-phthalamid)butyl]-piperazinhydrochlorid (NAN-190) und Ipsapiron gezeigt:

### 1. Methode: "Single trial passive avoidance" bei Ratten

In diesem Test wird die Fähigkeit von Ratten gemessen, sich an ein erlerntes Verhalten nach 48 Stunden zu erinnern. Es wird als eine Messung des Langzeitgedächtnisses interpretiert.

Am ersten Tag des Lernens werden die Tiere auf eine Plattform gesetzt. Wenn sie herunterspringen, bekommen sie einen kurzen Fußschock und werden sofort danach aus dem Apparat genommen. Zwei Tage später werden die Tiere wieder auf die gleiche Plattform gesetzt und die Zeit, die sie auf der Plattform bleiben, bevor sie herunterspringen, wird gemessen (bis maximal 300 s). Die verlängerte Zeit vor dem Herunterspringen spiegelt die Fähigkeit wieder, sich an die Straferfahrung des ersten Tages zu erinnern. Die Testsubstanz wurde 30 min vor der Lernphase verabreicht, um die Sicherheit, daß das verbesserte Lernen auf einen substanzvermittelten Effekt zurückzuführen ist, zu erhöhen. NAN-190 ist ein gemischter Agonist/Antagonist in unterschiedlichen in vivo und in vitro Testverfahren. 10 Tieren pro Gruppe wurden 1,0; 2,0 oder 4,0 mg/kg NAN-190, gelöst in 0,85 % NaCl, i.p. verabreicht. In einer Kontrollgruppe von 10 Tieren wurde nur Vehikel verabreicht.

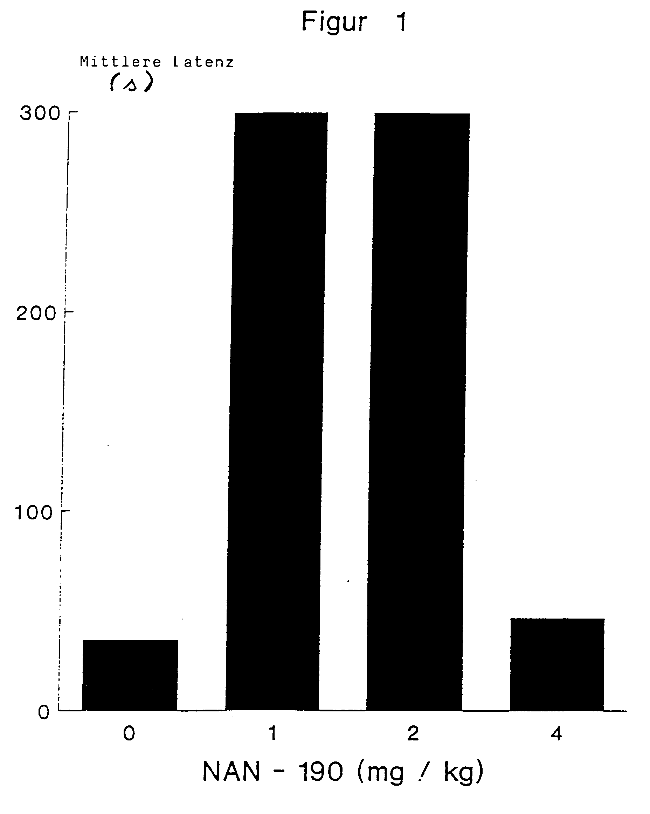

Die Ergebnisse zeigen, daß NAN-190 (1,0 und 2,0 mg/kg i.p., 30 min vor der Lernphase verabreicht) eine verbesserte Erinnerung des Fluchtverhaltens bewirkt, während 4,0 mg/kg NAN-190 keinen Effekt hat (Fig. 1). In der Lernphase zeigen die behandelten Tiere keine Unterschiede gegenüber den Kontrolltieren bei der Verzögerung des Herunterspringens von der Plattform, ein Hinweis, daß die Behandlung keine nicht-spezifischen Effekte auf Leistungsvariablen bewirkt hat.

Die Daten wurden statistisch analysiert mittels nichtparametrischer Informationsstatistik. Als Variable wurde die Anzahl der Tiere benutzt, die von der Plattform heruntergesprungen sind, verglichen mit der Anzahl der Tiere, die auf der Plattform geblieben sind für 300 s) (d.h. die die perfekte Einhaltung des Verhaltens gezeigt haben). Diese Analyse zeigte einen gesamten Effekt der Drogenbehandlung (2l=46, 78; df = 23;p < 0,001), weil mehr Tiere nach Behandlung mit 1,0 oder 2,0 mg/kg NAN-190 eine perfekte Einhaltung des Verhaltens gezeigt haben als nach Vehikelbehandlung. Es ergab sich kein erkennbarer Unterschied zwischen den Kontrolltieren und den mit 4,0 mg/kg NAN-190 behandelten Gruppen. Die Ergebnisse zeigen, daß die Behandlung mit NAN-190 1,0 mg/kg und 2,0 mg/kg ein verbessertes Lernen hervorgerufen hat.

### 2. Methode: Delayed non-matching to position bei Ratten

In diesem Test wird die Fähigkeit von Ratten gemessen, sich an eine räumliche Anordnung zu erinnern. Der Test wird als eine Messung des Kurzzeitgedächtnisses interpretiert.

Dazu werden Ratten in standardisierten sogenannten "operant chambers" darauf trainiert, sich an die räumliche Anordnung (rechter oder linker Hebel der Kammer) ihrer letzten Antwort zu erinnern und entsprechend zu reagieren. Richtige Antworten (matches) werden durch Gabe von Essen belohnt. Die zeitbedingte Verschlechterung des Gedächtnisses kann untersucht werden, indem der zeitliche Abstand zwischen Präsentation der Probe (rechter oder linker Hebel) variiert wird. Testsubstanzen, die keinen spezifischen Einfluß auf das Gedächtnis haben, beeinflussen die Genauigkeit der Antworten unabhängig von der Verzögerung, während Testsubstanzen, die speziell das Arbeitskurzzeitgedächtnis beeinflussen, eine Verschlechterung oder Verbesserung der Durchführung, die direkt von der zeitlichen Verzögerung abhängt, hervorrufen.

Im ersten Experiment wurde Ipsapirone HCl, gelöst in 0.85% NaCl, 30 min vor Testbeginn injiziert (i.p., n=12 Ratten). Alle Ratten wurden mit allen Dosierungen (0, 1, 3 und 10 mg/kg) getestet, die Dosierungen wurden entsprechend einem "Latin Square design" angelegt. Wenigstens 2 Probeläufe ohne Applikation von Testsubstanz wurden zur Separation der Testsubstanzprüfungen durchgeführt. Im zweiten Experiment wurde die Fähigkeit von Ipsapirone HCl untersucht, eine durch Scopolamin verursachte Beeinträchtigung aufzuheben. Dazu wurden Ratten (n=12) 45 min vor dem Testbeginn Scopolamin HCl (0.14 mg/kg, gelöst in 0.85% NaCl) i.p. verabreicht gefolgt von einer um 15 min verzögerten Gabe von Ipsapirone HCl (Dosierung und experimentelle Durchführung siehe oben).

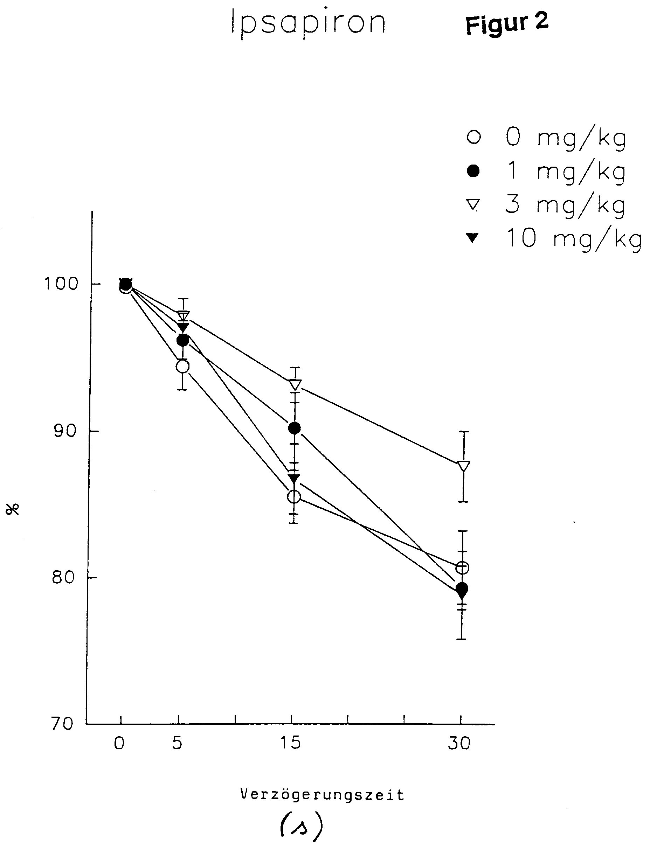

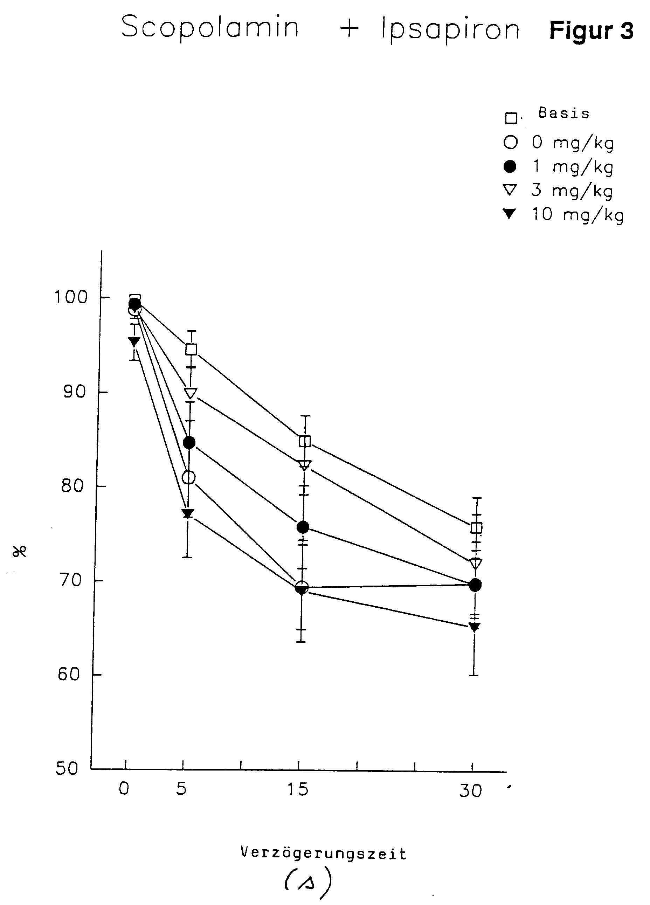

Die Ergebnisse von diesen Experimenten zeigen, daß Ipsapiron, wenn es allein verabreicht wird, die Leistung im "delayed matching" (siehe Beschreibung) verbessert (s. Fig. 2). Dies ist besonders auffällig bei den längeren Zeitabstähden (15 und 30 s). Scopolamingabe verursacht eine drastische Beeinträchtigung der Genauigkeit der Antworten, die direkt mit der Verzögerung im Zusammenhang steht. Dieser Effekt wurde vollständig antagonisiert durch die Gabe von Ipsapiron (3.0 mg/kg) (s. Fig. 3).

Die Daten wurden statistisch analysiert mittels einer faktoriellen Varianzanalyse mit den Faktor "delay time" und Dosis. Im zweiten Experiment zeigte die Analyse einen signifikanten Effekt in bezug auf die zeitliche Verzögerung, da die Genauigkeit der korrekten Antworten mit der Zeit abnahm ( F = 104.02, df = 3, 33, p < 0.001) und eine signifikante Wirkung bei der Dosierung ( F = 3.96, df = 3, 33, p < 0.001). Eine post hoc Analyse nach Newman Keul zeigte, daß die Ratten signifikant häufiger korrekte Antworten lieferten, wenn sie mit 3 mg/ kg Ipsapirone vorbehandelt wurden. Im dritten Experiment zeigte die statistische Analyse ebenfalls einen signifikanten Effekt in bezug auf die zeitliche Verzögerung, da die Häufigkeit der korrekten Antworten mit der Zeit abnahm (F= 55.647, df = 3, 33, p < 0.001) eine signifikante Wirkung bei der Dosierung (F = 4.91, df = 4, 44, p < 0.005) und eine signifikante Dosierung: Zeit Interaktion (F = 2.19, df = 12,132, p = 0.01). Eine Post hoc Analyse zeigte, daß Scopolamin die Genauigkeit der Antworten im Vergleich zum Kontroll-Level signifikant beeinträchtigt. Folgte der Scopolamin-Verabreichung eine Gabe von Ipsapiron (3 mg/kg) waren die Versuchsergebnisse in bezug auf Genauigkeit der Antworten nicht signifikant verschieden von den Kontrollversuchen, d.h. die Effekte von Scopolamin wurden völlig aufgehoben.

Diese Ergebnisse zeigen, daß Ipsapiron das Kurzzeitgedächnis verbessert und außerdem die Kognitions-störenden Wirkungen von Scopolamin (ein weitverbreitetes, pharmakologisches Mode der kognitiven Verluste in der SDAT) antagonisieren kann.

Aufgrund der Untersuchungsergebnisse bewirken die oben beschriebenen Verbindungen eine Verbesserung kognitiver Funktionen sowohl präventiv als auch therapeutisch analog Hydergin und Piracetam.

Die erfindungsgemäß verwendeten Arzneimittel werden nach an sich bekannten Verfahren hergestellt, indem man den Wirkstoff mit geeigneten Träger-, Hilfs- und/oder Zusatsstoffen in die Form eines pharmazeutischen Präparates bringt, das für die enterale oder parenterale Applikation geeignet ist. Die Applikation kann oral oder sublingual, als Feststoff in Form von Kapseln oder Tabletten oder als Flüssigkeit in Form von Lösungen, Suspensionen, Elixieren oder Emulsionen oder rektal in Form von Suppositorien oder in Form von gegebenenfalls auch subcutan anwendbaren Injektionslösungen erfolgen. Als Hilfsstoffe für die gewünschte Arzneimittelformulierung sind die dem Fachmann bekannten inerten organischen und anorganischen Trägermaterialien geeignet wie zum Beispiel Wasser, Gelatine, Gummi arabicum, Milchzucker, Stärke, Magnesiumstearat, Talk, pflanzliche Öle, Polyalkylenglykole usw.. Gegebenenfalls können darüber hinaus Konservierungs-, Stabilisierungs, Netzmittel, Emulgatoren oder Salze zur Verähderung des osmotischen Druckes oder Puffer enthalten sein.

Die pharmazeutischen Präparate können in fester Form zum Beispiel als Tabletten, Dragees, Suppositorien, Kapseln oder in flüssiger Form, zum Beispiel als Lösungen, Suspensionen oder Emulsionen vorliegen oder als Depotzubereitung formuliert sein..

Als Trägersysteme können auch grenzflächennahe Hilfsstoffe wie Salze der Gallensäuren oder tierische oder pflanzliche Phospholide, aber auch Mischungen davon sowie Liposome oder deren Bestandteile verwendet werden.

Für die orale Anwendung sind insbesondere Tablette, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,2 - 200 mg, wobei die Dosis als einmal zu verabreichende Einzeldosis oder unterteilt in 2 oder mehrere Tagesdosen gegeben werden kann. Im allgemeinen wird der Wirkstoff ein- bis viermal täglich appliziert, gegebenenfalls auch zusammen mit anderen therapeutischen Wirkstoffen. Die erfindungsgemäßen Arzneimittel enthalten im allgemeinen 0,01 bis 20 Gew. % des Wirkstoffes bezogen auf die Zubereitung.

## Patentansprüche

1. Verwendung von Antagonisten oder partiellen Agonisten am 5-HT₁ₐ-Rezeptor oder deren physiologisch verträgliche Salze zur Herstellung eines Arzneimittels zur Prävention und Behandlung von kognitiven Störungen.

2. Verwendung nach Anspruch 1, wobei es sich um kognitive Störungen handelt, die im höheren Lebensalter auftreten.

3. Verwendung nach Anspruch 1, wobei die kognitiven Störungen mit AIDS-abhängiger Demenz assoziiert sind.

4. Verwendung nach Anspruch 1, wobei die kognitiven Störungen mit Parkinson-abhängiger Demenz assoziiert sind.

5. Verwendung nach Anspruch 1, wobei die kognitiven Störungen bei Alzheimer-Krankheit auftreten.

6. Verwendung nach Anspruch 1-5, dadurch gekennzeichnet, daß Piperazinderivate als Wirkstoffe eingesetzt werden.

7. Verwendung nach Anspruch 1-6, dadurch gekennzeichnet, daß Ipsapiron als Wirkstoff eingesetzt wird.

8. Verwendung nach Anspruch 1-6, dadurch gekennzeichnet, daß NAN 190 als Wirkstoff eingesetzt wird.

## Claims

1. Use of antagonists or partial agonists on the 5-HT₁ₐ receptor or their physiologically tolerable salts for the production of a medicament for the prevention and treatment of cognitive disorders.

2. Use according to Claim 1, the cognitive disorders concerned being those which occur in relatively old age.

3. Use according to Claim 1, the cognitive disorders being associated with AIDS-dependent dementia.

4. Use according to Claim 1, the cognitive disorders being associated with Parkinson-dependent dementia.

5. Use according to Claim 1, the cognitive disorders being associated with Alzheimer-dependent dementia.

6. Use according to Claims 1-5, characterized in that piperazine derivatives are employed as active compounds.

7. Use according to Claims 1-6, characterized in that ipsapirone is employed as an active compound.

8. Use according to Claims 1-6, characterized in that NAN 190 is employed as an active compound.

## Revendications

1. Utilisation d'antagonistes ou d'agonistes partiels sur le récepteur 5-HT₁ₐ ou de leurs sels acceptables du point de vue physiologique pour la préparation d'un médicament destiné à la prévention et au traitement de troubles cognitifs.

2. Utilisation suivant la revendication 1, dans laquelle il s'agit de troubles cognitifs qui apparaissent à un stade avancé de vieillesse.

3. Utilisation suivant la revendication 1, dans laquelle les troubles cognitifs sont associés à la démence liée au SIDA.

4. Utilisation suivant la revendication 1, dans laquelle les troubles cognitifs sont associés à la démence liée à la maladie de Parkinson.

5. Utilisation suivant la revendication 1, dans laquelle les troubles cognitifs accompagnent la maladie d'Alzheimer.

6. Utilisation suivant les revendications 1 à 5, caractérisée en ce que des dérivés de pipérazine sont utilisés comme substances actives.

7. Utilisation suivant les revendications 1 à 6, caractérisée en ce que l'ipsapiron est utilisé comme substance active.

8. Utilisation suivant les revendications 1 à 6, caractérisée en ce que le NAN 190 est utilisé comme substance active.
